# EUROPÄISCHE PATENTANMELDUNG

(11) **EP 0 903 124 A2**
(43) Veröffentlichungstag der Anmeldung: **24.03.1999**
(21) Anmeldenummer: 98115045.1
(22) Anmeldetag: 11.08.1998
(51) Int. Cl.: A61F 2/28, A61C 8/00

(54) **Hilfsmittel zur Anregung des Zahnwachstums und zur Kieferregenerierung**

(30) Priorität: 19.09.1997 DE 19741273
(71) Anmelder: Flaum, Johann, 58509 Lüdenscheid (DE)
(72) Erfinder: Flaum, Johann, 58509 Lüdenscheid (DE)
(74) Vertreter: Hassler, Werner, Dr.

(57) **Zusammenfassung**

Ein Hilfsmittel zur Anregung des Zahnwachstums und zur Kieferregenerierung, wobei Seitenteile des Hilfsmittels am Zahnfleisch anliegen. Das technische Problem ist es dagegen, die Regenerierung ohne entsprechend fixierte Knochenmaterialien zu ermöglichen. Die Innenseiten der Seitenteile (6) sind Nadeln (1) besetzt.

## Beschreibung

Die Erfindung betrifft ein Hilfsmittel zur Anregung des Zahnwachstums und zur Kieferregenerierung, wobei Seitenteile des Hilfsmittels am Zahnfleisch anliegen.

Derartige Hilfsmittel zur Kieferregenerierung sind aus der DE 43 02 709 C1 und DE 91 15 341 U1 bekannt, wobei in beiden Fällen Seitenteile am Zahnfleisch anliegen, die Knochenaufbaumaterial an der zu regenerierenden Knochenstelle fixieren.

Aufgabe der Erfindung ist es dagegen, die Regenerierung ohne entsprechend fixierte Knochenmaterialien zu ermöglichen.

Diese Aufgabe wird nachd der Erfindung dadurch gelöst, daß die Innenseiten der Seitenteile (6) mit Nadeln (1) besetzt sind.

Die Erfindung unterscheidet sich insofern vom Stand der Technik, als durch kissenartig angeordnete Nadeln eine Aktivierung des Zahnfleischs und des Kiefers bewirkt wird. Dadurch können Restkeime von Zähnen zum Wachstum angeregt werden. Langzeitig ist eine dauerhafte Sanierung möglich.

Eine intensive Wirkung wird dadurch erzielt, daß die Länge der Nadeln ca. 1 mm beträgt.

Besonders günstig hat sich erwiesen, daß der gegenseitige Abstand der Nadeln ca. 1 mm beträgt.

Eine einfache Herstellung des Hilfsmittels wird dadurch garantiert, daß die Nadeln einstückig mit den Seitenteilen aus einem zahnprothetischen Werkstoff ausgebildet sind.

Ausführungsbeispiele der Erfindung werden anhand der Zeichnungen näher erläutert. Es zeigen.
Fig. 1 einen Schnitt durch ein erstes Ausführungsbeispiel,
Fig. 2 eine Draufsicht aufdieses Ausführungsbeispiel,
Fig. 3 ein zweites Ausführungsbeispiel und
Fig. 4 eine Abwandlung des zweiten Ausführungsbeispiel.

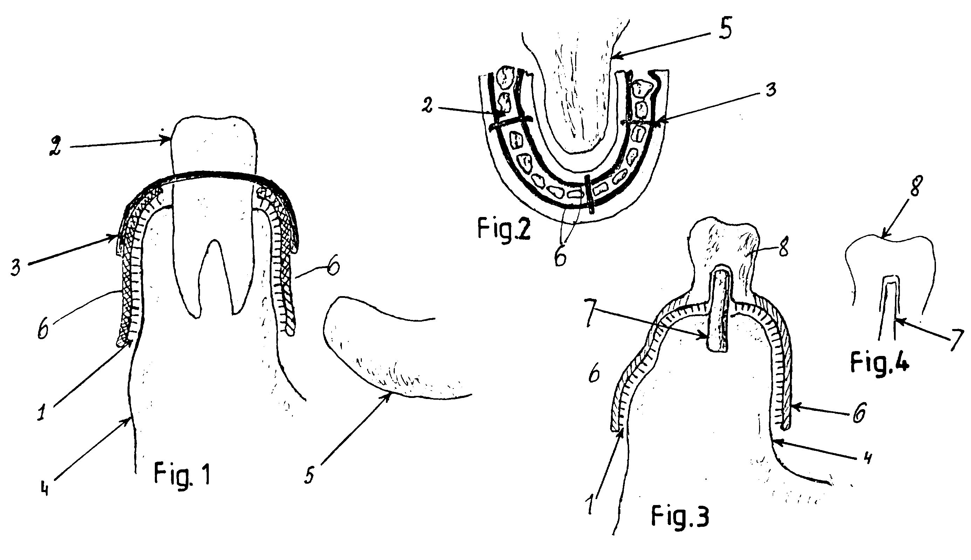

Fig. 1 zeigt einen Schnitt durch den Kiefer 4 mit einem Zahn 2. Der Kiefer 4 ist mit Zahnfleisch bedeckt, was im Einzelnen nicht erläutert ist. Der Zahn 2 oder Restzahn dient zur Verankerung der Klammern 3 des Hilfsmittels, dessen Seitenteile 6 durch die Klammern 3 zusammengehalten und zusammengespannt sind. Die Seitenteile 6 sind auf der jeweiligen Innenseite mit Nadeln 1 besetzt. Die Seitenteile 6 bedecken die gesamte Fläche des Zahnfleischs. Im Mundraum befindet sich dieZunge 5.

Die Nadeln 1 haben eine Länge von ca. 1 mm und einen gegenseitigen Abstand von ca. 1 mm. Die Nadeln 1 sind einstückig mit den Seitenteile 6 des Hilfsmittels aus einem zahnprothetischen Werkstoff ausgebildet. Sie können auch aus einem anderen verträglichen Werkstoff bestehen. Sie müssen eine ausreichende Haltbarkeit aufweisen, insbesondere müssen die Spitzen bruchsicher sein. In Abhängigkeit von den anatomischen Verhältnissen und dem Behandlungsverlauf kann man auch eine andere Anordnung der Nadeln wählen.

Das Hilfsmittel mit den Nadeln 1 muß über längere Zeit getragen werden Die Nadeln 1 regen die Durchblutung des Zahnfleischs und des Kieferknochens an. Sie aktivieren noch vorhandene Zahnkeime, so daß in vielen Fällen ein Zabnwachstum und eine Regenerierung des Kieferknochens erreicht wird. Die Behandlung kann sich durchaus über mehrere Jahre erstrecken. Durch das Kauen wird die Wirkung der Nadeln gefördert.

Fig. 3 zeigt ein Hilfsmittel, bei dem ein Zahn 8 aufeinen im Kiefer verankerten Stift 7 aufgesetzt ist. Der Zahn 8 hält die Seitenteile 6. Der Stift 7 kann nach Fig. 4 auch ein Restzahn sein.

## Patentansprüche

1. Hilfsmittel zur Anregung des Zahnwachstums und zur Kieferregenerierung, wobei Seitenteile des Hilfsmittels am Zahnfleisch anliegen, dadurch gekennzeichnet, daß die Innenseiten der Seitenteile (6) mit Nadeln (1) besetzt sind.

2. Hilfsmittel nach Anspruch 1, dadurch gekennzeichnet, daß die Länge der Nadeln (1) ca. 1 mm beträgt.

3. Hilfsmittel nach Anspruch 1 und 2, dadurch gekennzeichnet, daß der gegenseitige Abstand der Nadeln (1) ca. 1 mm beträgt.

4. Hilfsmmittel nach einem der Ansprüche 1 bis 3, dadurch gekennzeichnet, daß die Nadeln (1) einstückig mit den Seitenteilen (6) aus einem zahnprothetischen Werkstoff ausgebildet sind.
